# EUROPEAN PATENT SPECIFICATION

(11) **EP 3 824 821 B1**
(45) Date of publication and mention of the grant of the patent: **17.01.2024**
(21) Application number: 20208319.2
(22) Date of filing: 18.11.2020
(51) Int. Cl.: A61B 17/12, A61M 25/10, A61B 17/22, A61B 17/00, A61B 17/3207

(54) **CATHETER FOR ISOLATED INTRAVASCULAR TREATMENT WITH PERFUSION BYPASS**
KATHETER FÜR ISOLIERTE INTRAVASKULÄRE BEHANDLUNG MIT PERFUSIONSBYPASS
CATHÉTER POUR TRAITEMENT INTRAVASCULAIRE ISOLÉ AVEC BYPASS DE PERFUSION

(30) Priority: 19.11.2019 US 201916688111
(43) Date of publication of application: 26.05.2021
(73) Proprietor: Neuravi Limited, Galway H91 K5YD (IE)
(72) Inventor: STEFANOV, Petrica, Galway, H91 K5YD (IE)
(74) Representative: Carpmaels & Ransford LLP

(56) References cited:
- WO-A1-89/00829
- WO-A1-95/11719
- WO-A1-98/08558
- WO-A1-2011/012575
- WO-A1-2018/106788
- US-A- 5 222 941
- US-A- 5 256 141
- US-A1- 2005 273 050

## Description

### Field of Invention

The present invention generally relates to medical devices ts for treating intravascular lesions, and more specifically to a catheter for treatments of lesions relating to intracranial atherosclerosis disease (ICAD).

### Background

Atherosclerosis results from lesions which narrow and reduce the space in the lumen of a blood vessel. Such lesions are usually composed of plaque, which can be fat, cholesterol, calcium, or other components of the blood. Severe occlusion or closure can impede the flow of oxygenated blood to different organs and parts of the body and result in cardiovascular disorders such as heart attack or stroke. Narrowing of vessels, or stenosis, increases the risk that clots and other emboli can lodge at such locations, especially in the neurovascular where vessel diameters are already small. ICAD is the narrowing of those arteries and vessels supplying blood to the brain and represents the most common proximate mechanism of ischemic stroke.

Treatment for vascular occlusions can include utilizing drugs, such as anticoagulants or anti-platelet agents, as well as medical procedures such as surgical endarterectomy, angioplasty, and stenting. A physician may select one or more treatment methods based on the difficulty of administering the treatment, potential effectiveness of the treatment, and risks associated with the treatment. Factors affecting these considerations can include the degree of stenosis, the shape of the target treatment site (i.e. truncal, branching, etc.), accessibility of the target treating site, and the patient's overall condition. For intravascular drug delivery, migration of drugs from the treatment site can case the treatment at the site to be less effective and/or adversely affect vascular to which the drug migrates. Mechanical treatments can cause the lesion to rupture or fragment. Such fragments can include but are not limited to blood clots, plaque, and other thrombi debris. The fragments can lead to vascular occlusions causing extensive stroke or death. Particularly in the case of ICAD, prolonging treatment can increase the likelihood that the lesion ruptures and/or can prolong arrest of blood flow to neurovasculature resulting in irreversible brain damage.

Applicants therefore recognize a need for systems and devices to continue to address and improve treatments for intravascular occlusions, specifically ICAD.

US 2005/273050 A1 relates to a catheter and a method for infusion therapy that can transluminally deliver drugs or the like to a target spot without invading areas other than a diseased area. The catheter is a balloon catheter for insertion into a blood vessel in which four lumens extending along an axis are formed in one catheter body, and two balloons are arranged axially in parallel. The plurality of lumens include: an infusion lumen that communicates with an infusion hole between the two balloons; balloon lumens that communicate with insides of the two balloons to expand the balloons; and a guide lumen that also serves as a bypass lumen.

US 5256141 A relates to a pair of spaced inflatable balloons that define proximal and distal occlusion zones for temporarily occluding normal vascular blood flow in the vascular segment during deployment of the biologically active materials. A flow bypass lumen connects proximal and distal infusion port zones flanking the occlusion zones and maintaining blood flow about and beyond the vascular segment during occlusion.

WO 95/11719 A1 relates to a catheter dimensioned to be insertable into the body passage and having a lateral wall and a distal end via which the catheter can be inserted into the body passage, the catheter being provided internally with fluid conducting passages and a fluid outlet passage which extends through the lateral wall and communicates with one of the fluid conducting passages; and first and second balloons carried by the catheter and extending outwardly from the lateral wall.

WO 2018/106788 A1 relates to an inter- and intra-catheter flow device for the management of vascular bleeding disorders that provides a liquid flow-pass between proximal and distal balloons for bridging the circulation between the upper and lower segments of a hemorrhaging artery or blood vessel, while blocking the blood flow to the hemorrhaging middle segment(s) of the artery or blood vessel between the two or more balloons.

### Summary

The invention is defined in the independent claim.

Embodiments are defined in the dependent claims.

### Brief Description of the Drawings

The above and further aspects of this invention are further discussed with reference to the following description in conjunction with the accompanying drawings, in which like numerals indicate like structural elements and features in various figures. The drawings are not necessarily to scale, emphasis instead being placed upon illustrating principles of the invention. The figures depict one or more implementations of the inventive devices, by way of example only, not by way of limitation.
FIG. 1A is an illustration of a system for intravascular treatment at a treatment site during a treatment method according to aspects of the present disclosure;
FIG. 1B is an illustration of a proximal end of the system for intravascular treatment according to aspects of the present disclosure;
FIG. 2A is an illustration of an intravascular treatment system placed across a lesion according to aspects of the present disclosure;
FIG. 2B is an illustration of the intravascular treatment system of FIG. 2B placed across the lesion indicating cross-sectional views according to aspects of the present disclosure;
FIGs. 3A through 3H are illustrations of cross-sectional views as indicated in FIG. 2B according to aspects of the present disclosure;
FIG. 4A is an illustration of another system for intravascular treatment at a treatment site during a treatment method according to aspects of the present disclosure;
FIG. 4B is an illustration of the system illustrated in FIG. 4A placed across the lesion indicating cross-sectional views according to aspects of the present disclosure;
FIGs. 5A through 5G are illustrations of cross-sectional views as indicated in FIG. 4B according to aspects of the present disclosure;
FIG. 6A is an illustration of another system for intravascular treatment at a treatment site during a treatment method according to aspects of the present disclosure;
FIGs. 6B through 6D are cross sectional views of the system illustrated in FIG. 6A according to aspects of the present disclosure;
FIGs. 7A through 7C are illustrations of the system illustrated in FIGs. 6A through 6D during steps of a method of treatment according to aspects of the present disclosure;
FIGs. 8A and 8B are illustrations of another system for intravascular treatment functioning according to the principles of the system illustrated in FIGs. 6A through 6D and FIGs. 7A through 7C according to aspects of the present disclosure;
FIGs. 9A through 9G are illustrations of steps of treatment using an intravascular treatment system such as one of the intravascular treatment systems illustrated herein according to aspects of the present disclosure; and
FIG. 10 is a flow diagram outlining method steps of a method of intravascular treatment according to aspects of the present disclosure.

### Detailed Description

Example systems presented herein can have the benefit of isolating a targeted area of stenosis for diagnostics, drug administration, aspiration, and/or restoration of blood perfusion. The system can include two compliant balloons. Alternatively, the system can include self-expandable and/or otherwise expandable occlusion devices in place of, or in addition to, one or both of the two compliant balloons. Example systems presented herein can be used for various treatments. In treatment methods including diagnostics, contrast medium can be retained at the targeted area for enhanced visualization and/or reduced quantity of contrast medium. In treatment methods including drug administration, drugs can be administered locally through a skive or other device opening positioned between the occlusion devices. Drugs can be administered in the stenosis area for a specific time with reduced risk of drugs migrating. Drugs can be aspirated through the same opening as the drugs were administered. In treatment methods including aspiration, the same or a different skive or device opening can be used to remove plaque ruptures that might occur during the procedure. During diagnostics, drug administration, and/or aspiration, blood perfusion can be restored by flowing blood through a skive or other device opening positioned in the proximal direction in relation to the expandable occlusion devices, through a tube extending across the target treatment area, and out of a distal device opening positioned in the distal direction in relation to the expandable occlusion devices.

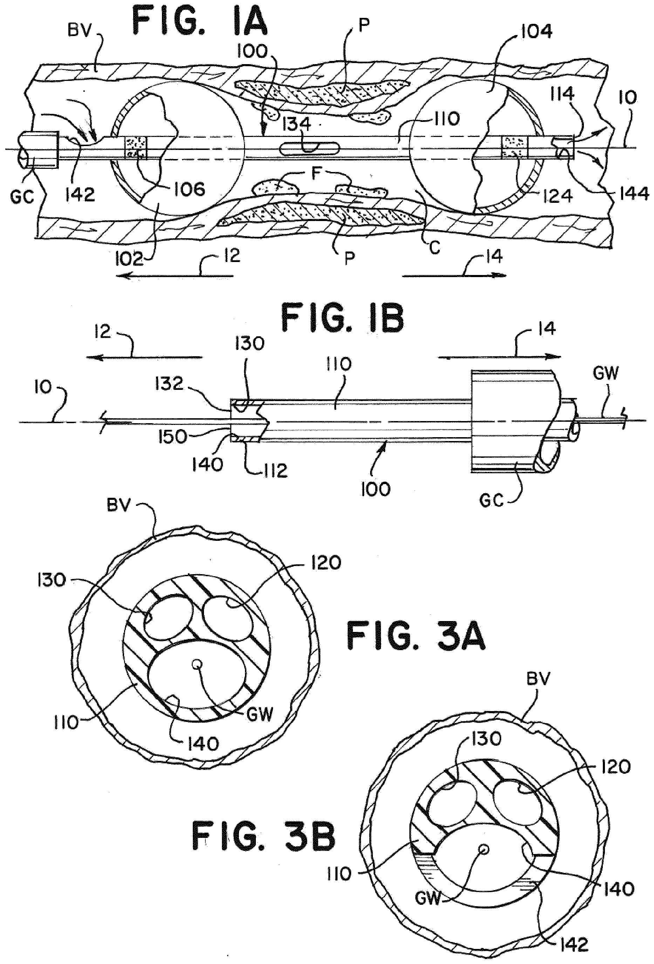

FIGs. 1A and 1B illustrate an example system for intravascular treatment. FIG. 1A illustrates a distal portion of the system positioned at a target treatment area within a blood vessel BV. FIG. 1B illustrates a proximal portion of the system that can be manipulated by a user during a treatment procedure. The system can include a device 100 having a tubular body 110. The device can function as a catheter 100 having a length measurable from a proximal end 112 of the tubular body 110 (FIG. 1B) to a distal end 114 of the tubular body (FIG. 1A). The length can be sized to extend from outside a patient, into a blood vessel (e.g. through the femoral artery), through vasculature to an intravascular blood vessel (e.g. in neurovasculature). The device 100 can include a proximal expandable occluding element 102 disposed on the tubular body 110. The device 100 can include a distal expandable occluding element 104 disposed on the tubular body 110. The distal expandable occluding element 104 can be disposed in a distal direction 14 in relation to the proximal expandable occluding element 102. The device can include a treatment lumen 130 having a proximal opening 132 near the proximal end 112 of the tubular body 110 and a distal opening 134 positioned near the distal end 114 of the tubular body 110. The distal opening 134 of the treatment lumen 130 can be positioned between the occluding elements 102, 104. The device 100 can include a perfusion lumen 140 having a proximal opening 142 positioned in a proximal direction 12 in relation to the occluding elements 102, 104 and a distal opening 144 positioned in the distal direction 14 in relation to the occluding elements 102, 104.

The device 100 can be delivered to the treatment site by means as appreciated and understood by a person of ordinary skill in the art. The device 100 can be delivered through a guide catheter GC as illustrated in FIGs. 1A and 1B or delivered without a guide catheter GC. The device 100 can be guided over a guide wire GW. The device 100 can be guided over an elongated member extending through a lumen of the device to facilitate placement of the device at a treatment site in a manner similar to known methods for delivering a catheter over an elongated member. The elongated member need not have a solid core. Such elongated members are referred to herein generically as a "guide wire." Alternatively, the device 100 need not be delivered over a guide wire.

The proximal expandable occluding element 102 can be expandable from a collapsed shape sized to traverse the guide catheter GC to an expanded shape sized to occlude the blood vessel BV. The distal expandable occluding element 102 can be expandable from a collapsed shape sized to traverse the guide catheter GC to an expanded shape sized to occlude the blood vessel BV. One or both of the proximal and distal occluding elements 102, 104 can include a fluidically impermeable material. Preferably both occluding elements 102, 104 include fluidically impermeable material and are configured to block blood flow through the blood vessel BV when expanded such that the perfusion lumen 140 is the sole path for blood to flow across the treatment site. One of the proximal and distal occluding elements 102, 104 can include balloon expandable via an inflation lumen. Additionally, or alternatively, one of the proximal and distal occluding elements 102, 104 can include a self-expandable element. The proximal and distal occluding elements 102, 104 can each be expandable to appose the blood vessel BV and can be effective to arrest flow of blood through blood vessel BV outside of the confines of the device 100. In other words, the proximal and distal occluding elements 102, 104 can be expanded to define an isolated treatment region within the blood vessel BV extending between the two occluding elements 102, 104.

The treatment lumen 130 can be configured to allow fluids to be delivered between the proximal opening 132 near the proximal end 112 of the elongated tubular body 110 and the distal opening 134 between the occluding elements 102, 104. Additionally, or alternatively, the treatment lumen 130 can be configured to allow mechanical treatment devices to extend from the proximal opening 132 to the distal opening 134. During a treatment, the proximal opening 132 can be accessible by a user (e.g. physician) to aspirate, inject fluids, and/or manipulate mechanical treatment devices while the distal opening 134 is positioned at a treatment site (e.g. near a lesion P in a blood vessel BV). The aspiration, fluid injection, and or mechanical treatment device manipulation via the treatment lumen 130 can occur while the proximal and distal occlusion elements 102, 104 are expanded to appose the blood vessel BV. The treatment lumen 130 can be a delivery lumen sized for delivery of a drug suitable for treating the intravascular lesion P. The treatment lumen 130 can further be coated with a coating effective to inhibit adherence of a sealant effective to seal an intravascular lesion P. Additionally, or alternatively, the treatment lumen 130 can be sized to aspirate blood from the blood vessel BV. The treatment lumen 130 can further include sufficient structural integrity to withstand collapsing due to suction produced during aspiration. Additionally, or alternatively, the treatment lumen 130 can be sized to accommodate a clot retriever, a mechanical treatment device configured to disrupt plaque, and/or a mechanical treatment device configured to capture plaque fragments.

The perfusion lumen 140 can serve as a bypass lumen configured to allow blood to flow through the proximal and distal occlusion elements 102, 104. During a treatment, blood can flow between the proximal opening 142 of the perfusion lumen and the distal opening 144 of the perfusion lumen 140 to at least partially allow blood perfusion. The perfusion lumen 140 can further serve as a guide lumen configured to allow passage of a guide wire GW from a proximal end 112 of the tubular body 110 to the distal end 114 of the tubular body 110. Configured as such, the perfusion lumen 140 of the device 100 can slide over the guide wire GW during delivery to the treatment site. Alternatively, the device 100 can include a guide wire lumen separate from the perfusion lumen 140, in which case the perfusion lumen 140 need not extend in the proximal direction beyond the proximal opening 142.

The device 100 can further include radiopaque materials to aid in placement of the device 100 during treatment. As illustrated in FIG. 1A, the device 100 can include a proximal marker band 106 positioned on the tubular body 110 near the proximal occluding element 102 and a distal marker band 124 positioned on the tubular body 110 near the distal occluding element 104. Radiopaque material can be positioned or otherwise incorporated as appreciated and understood by a person of ordinary skill in the art.

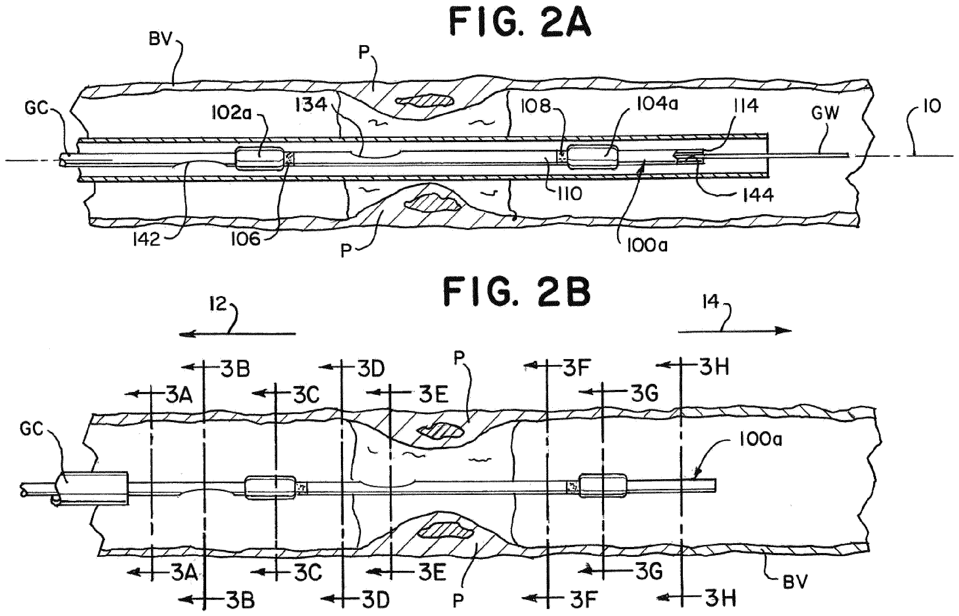

FIGs. 2A and 2B are illustrations of an intravascular treatment system in a collapsed configuration, placed across a lesion P. FIGs. 3A through 3H are illustrations of cross-sectional views of the system as indicated in FIG. 2B. Referring collectively to FIGs. 2A, 2B, and 3A through 3H, the system can include a treatment device 100a, a guide catheter GC, and a guide wire GW such as illustrated and described in relation to FIGs. 1A and 1B. The treatment device 100a illustrated in FIGs. 2A, 2B, and 3A through 3H is illustrated as including a proximal expandable element 102a that includes a balloon and a distal expandable element 104a that includes a balloon. The treatment device 100a can otherwise include an elongated body 110, a treatment lumen 130, and a perfusion lumen 140 as illustrated and/or described in relation to FIGs. 1A and 1B.

FIG. 2A illustrates the device 100a sheathed by a guide catheter GC while placed across the lesion P. Alternatively, the device 100a can be configured to be delivered across the lesion P unsheathed. In some treatments, the device 100a can be delivered across the lesion P unsheathed. Whether delivered sheathed or unsheathed, the device 100a can be positioned unsheathed as illustrated in FIG. 2B. The device 100a can extend along a longitudinal axis 10 as indicated in FIG. 2B. The cross sections illustrated in FIGs. 3A through 3H are viewed looking in the proximal direction on the longitudinal axis 10.

FIG. 3A illustrates a cross section of the device 100a as indicated in FIG. 2B. The elongated tubular body 110 can include at least a portion of the treatment lumen 130. The elongated tubular body 110 can include at least a portion of the perfusion lumen 140. The perfusion lumen 140 can further provide a passage way for the guide wire GW as illustrated. Configured as such, the perfusion lumen 140 can include a third opening 150 near the proximal end 112 of the device 100a (See FIG. 1B) to allow the guide wire GW to enter the device 100a. Alternatively, the device 100a can include an additional lumen to allow passage of the guide wire GW; in which case the perfusion lumen 140 need not extend in the proximal direction 12 in relation to the proximal opening 142. The device 100a can include one or more inflation lumens 120 extending a majority of the length of the tubular body 110. As illustrated in FIGs. 3A through 3H, the device 100a can include a singular inflation lumen 120 in communication with both the proximal expandable element 102a balloon and the distal expandable element 104a balloon. Alternatively, the device 100a can include two separate inflation lumens - one for the proximal expandable element 102a balloon and one for the distal expandable element 104a balloon. Alternatively, only one of the proximal and distal expandable elements 102a, 104a can include a balloon and the device 100a can include a single inflation lumen for inflating the balloon.

FIG. 3B illustrates a cross section of the device 100a as indicated in FIG. 2B. The tubular body 110 can be cut away into the perfusion lumen 140 to create the proximal opening 142 of the perfusion lumen 140. The proximal opening 142 of the perfusion lumen 140 can be a skive in the side wall of the tubular body 110.

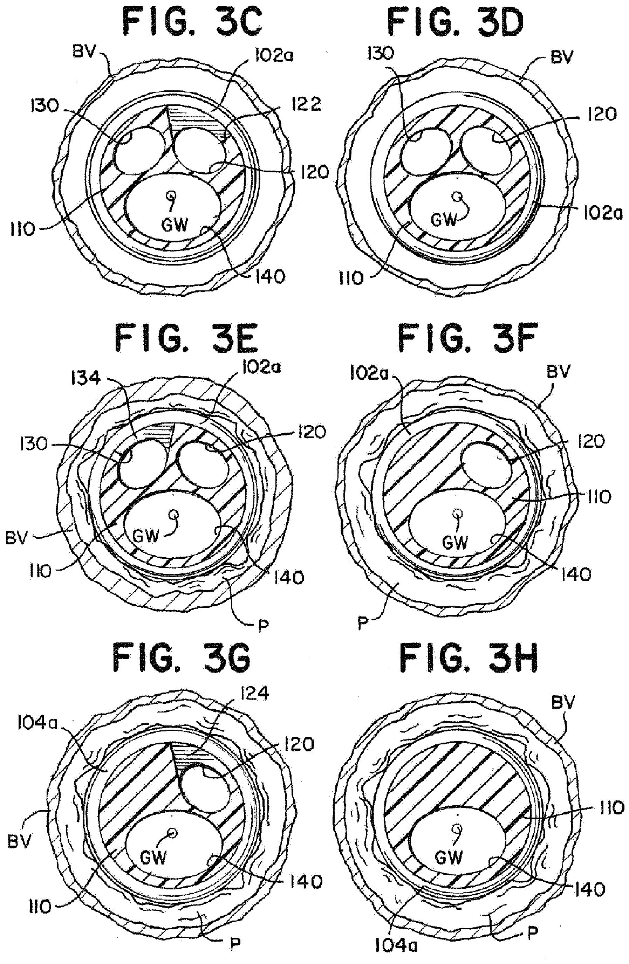

FIG. 3C illustrates a cross section of the device 100a as indicated in FIG. 2B. The balloon of the proximal expandable element 102a is illustrated surrounding the tubular body 110. The tubular body 110 can have an opening 122 under the balloon of the proximal expandable element 102a to allow the balloon to be inflated via the inflation lumen 120.

FIG. 3D illustrates a cross section of the device 100a as indicated in FIG. 2B. The inflation lumen 120 can extend in the distal direction 14 in relation to the balloon of the proximal expandable element 102a.

FIG. 3E illustrates a cross section of the device 100a as indicated in FIG. 2B. The tubular body 110 can include an opening 134 to the treatment lumen 130. As illustrated, during treatment, the opening 134 to the treatment lumen 130 can be placed at the lesion P. The opening 134 to the treatment lumen 130 can include a skive in the tubular body 110.

FIG. 3F illustrates a cross section of the device 100a as indicated in FIG. 2B. As illustrated, the treatment lumen 130 need not extend beyond the opening 134 positioned between the occluding elements 102a, 104a.

FIG. 3G illustrates a cross section of the device 100a as indicated in FIG. 2B. The tubular body 110 can include an opening 124 to the inflation lumen 120. The opening 124 to the inflation lumen 120 can be positioned under the balloon of the distal occluding element 104a. The balloon of the distal occluding element 104a can be inflated via the inflation lumen 120 through the opening 124 under the balloon.

FIG. 3H illustrates a cross section of the device 100a as indicated in FIG. 2B. The perfusion lumen 140 can extend in the distal direction 14 in relation to the distal occluding element 104a. The inflation lumen 120 need not extend in the distal direction 14 in relation to the distal occluding element 104a.

As illustrated in FIGs. 3A through 3H, the perfusion lumen 140 can provide a contiguous flow path from its proximal opening 142, through the first expandable occluding element 102a, through the second expandable occluding element 104a, and to the distal opening 144 of the perfusion lumen 140. The perfusion lumen 140 can be sized to facilitate blood perfusion.

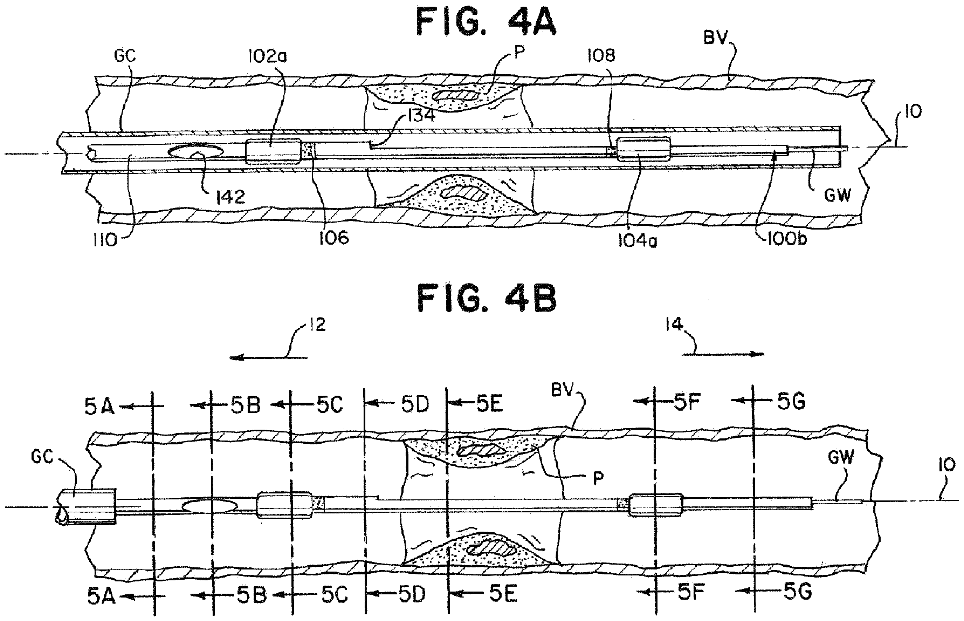

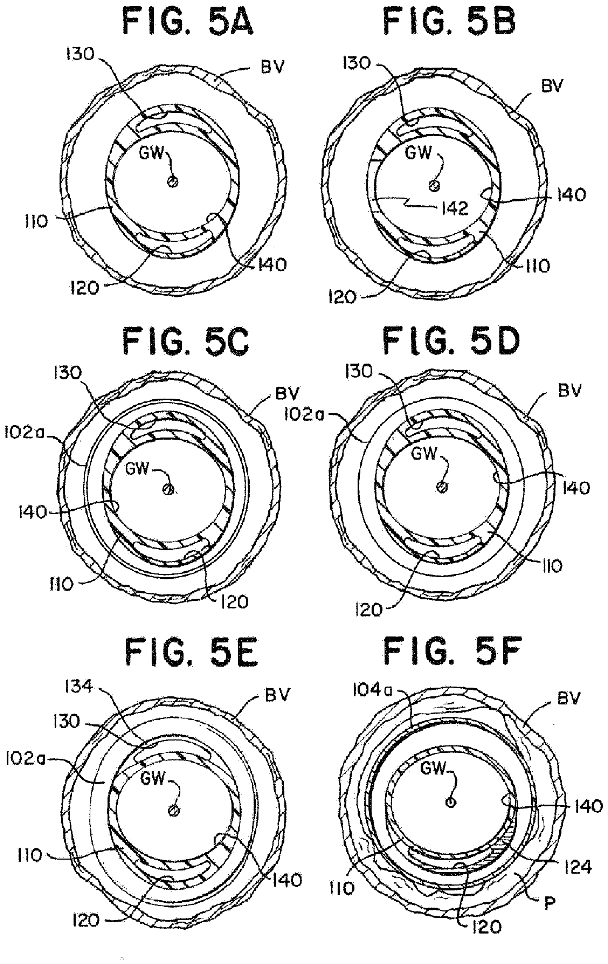

FIGs. 4A and 4B are illustrations of another intravascular treatment system in a collapsed configuration, placed across a lesion P. FIGs. 5A through 5G are illustrations of cross-sectional views of the system as indicated in FIG. 4B. Referring collectively to FIGs. 4A, 4B, and 5A through 5G, the system can include a treatment device 100b, a guide catheter GC, and a guide wire GW such as illustrated and described in relation to FIGs. 1A and 1B. The treatment device 100b illustrated in FIGs. 4A, 4B, and 5A through 5G is illustrated as including a proximal expandable element 102a that includes a balloon and a distal expandable element 104a that includes a balloon. The treatment device 100b can otherwise include an elongated body 110, a treatment lumen 130, and a perfusion lumen 140 as illustrated and described in relation to FIGs. 1A and 1B. The device 100b illustrated in FIGs. 4A, 4B, and 5A through 5G can have functionality similar to the device 100a illustrated in FIGs. 2A, 2B, and 3A through 3H. The device 100b illustrated in FIGs. 4A, 4B, and 5A through 5G differs from the device 100a illustrated in FIGs. 2A, 2B, and 3A through 3H primarily in the shape and position of the treatment lumen 130, perfusion lumen 140, and inflation lumen 120. The device 100b illustrated in FIGs. 4A, 4B, and 5A through 5G can include radiopaque markers 106, 108.

FIG. 4A illustrates the device 100b sheathed by a guide catheter GC while placed across the lesion P. Alternatively, the device 100b can be configured to be delivered across the lesion P unsheathed. In some treatments, the device 100b can be delivered across the lesion P unsheathed. Whether delivered sheathed or unsheathed, the device 100b can be positioned unsheathed as illustrated in FIG. 4B. The device 100b can extend along a longitudinal axis 10. The cross sections illustrated in FIGs. 5A through 5G are viewed looking in the proximal direction on the longitudinal axis 10.

FIG. 5A illustrates a cross section of the device 100b as indicated in FIG. 4B. The elongated tubular body 110 can include at least a portion of the treatment lumen 130. The elongated tubular body 110 can include at least a portion of the perfusion lumen 140. The perfusion lumen 140 can further provide a passage way for the guide wire GW as illustrated. Configured as such, the perfusion lumen 140 can include a third opening 150 near the proximal end 112 of the device 100b (See FIG. 1B) to allow the guide wire GW to enter the device 100b. Alternatively, the device 100b can include an additional lumen to allow passage of the guide wire GW; in which case the perfusion lumen 140 need not extend in the proximal direction 12 in relation to the proximal opening 142. The device 100b can include one or more inflation lumens 120 extending a majority of the length of the tubular body 110. As illustrated in FIGs. 5A through 5G, the device 100b can include a singular inflation lumen 120 in communication with both the proximal expandable element 102a balloon and the distal expandable element 104a balloon. Alternatively, the device 100b can include two separate inflation lumens - one for the proximal expandable element 102a balloon and one for the distal expandable element 104a balloon. Alternatively, only one of the proximal and distal expandable elements 102a, 104a can include a balloon and the device 100a can include a single inflation lumen for inflating the balloon.

FIG. 5B illustrates a cross section of the device 100b as indicated in FIG. 4B. The tubular body 110 can be cut away into the perfusion lumen 140 to create the proximal opening 142 of the perfusion lumen 140. The proximal opening 142 of the perfusion lumen 140 can be a skive in the side wall of the tubular body 110.

FIG. 5C illustrates a cross section of the device 100b as indicated in FIG. 4B. The balloon of the proximal expandable element 102a is illustrated surrounding the tubular body 110. The tubular body 110 can have an opening 122 under the balloon of the proximal expandable element 102a to allow the balloon to be inflated via the inflation lumen 120.

FIG. 5D illustrates a cross section of the device 100b as indicated in FIG. 4B. The inflation lumen 120 can extend in the distal direction 14 in relation to the balloon of the proximal expandable element 102a.

FIG. 5E illustrates a cross section of the device 100b as indicated in FIG. 4B. The tubular body 110 can include an opening 134 to the treatment lumen 130. As illustrated, during treatment, the opening 134 to the treatment lumen 130 can be placed at the lesion P. The opening 134 to the treatment lumen 130 can include a skive in the tubular body 110. As illustrated, the perfusion lumen 140 can be substantially circular in cross section, and the treatment lumen 130 can have a curved shape positioned around a portion of the perimeter of the perfusion lumen 140. Configured as such, in the distal direction 14 in relation to the opening 134 to the treatment lumen 130, the tubular body 110 can have a smaller outer circumference compared to the outer circumference of the tubular body 110 in the proximal direction in relation to the opening 134.

FIG. 5F illustrates a cross section of the device 100b as indicated in FIG. 4B. The tubular body 110 can include an opening 124 to the inflation lumen 120. The opening 124 to the inflation lumen 120 can be positioned under the balloon of the distal occluding element 104a. The balloon of the distal occluding element 104a can be inflated via the inflation lumen 120 through the opening 124 under the balloon. As illustrated, the inflation lumen 120 can have a curved shape positioned around a portion of the perimeter of the perfusion lumen 140. Configured as such, in the distal direction 14 in relation to the opening 124 to the inflation lumen 120, the tubular body 110 can have a smaller outer circumference compared to the outer circumference of the tubular body 110 in the proximal direction in relation to the opening 124.

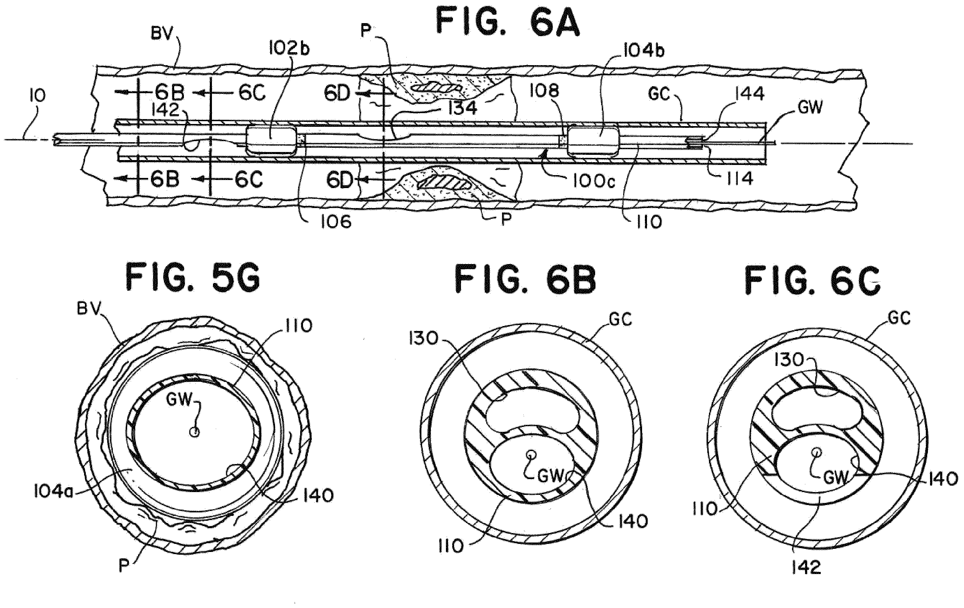

FIG. 5G illustrates a cross section of the device 100b as indicated in FIG. 4B. The perfusion lumen 140 can extend in the distal direction 14 in relation to the distal occluding element 104a. The inflation lumen 120 need not extend in the distal direction 14 in relation to the distal occluding element 104a.

As illustrated in FIGs. 5A through 5G, the perfusion lumen 140 can provide a continuous flow path from its proximal opening 142, through the first expandable occluding element 102a, through the second expandable occluding element 104a, and to the distal opening 144 of the perfusion lumen 140. The perfusion lumen 140 can be sized to facilitate blood perfusion.

FIG. 6A is an illustration of another intravascular treatment system in a collapsed configuration, placed across a lesion P. FIGs. 6B through 6D are illustrations of cross-sectional views of the system as indicated in FIG. 6A. Referring collectively to FIGs. 6A through 6D, the system can include a treatment device 100c, a guide catheter GC, and a guide wire GW such as illustrated and described in relation to FIGs. 1A and 1B. The treatment device 100c illustrated in FIGs. 6A through 6D is illustrated as including a proximal expandable element 102b that is self-expandable upon unsheathing and a distal expandable element 104b that is self-expandable upon unsheathing. The device 100c can be delivered across the lesion P while sheathed in the guide catheter GC or otherwise sheathed as appreciated and understood by a person of ordinary skill in the art. The treatment device 100c can include an elongated body 110, a treatment lumen 130, and a perfusion lumen 140 as illustrated and described in relation to FIGs. 1A and 1B. The device 100c illustrated in FIGs. 6A through 6D can include radiopaque markers 106, 108.

FIG. 6B illustrates a cross section of the device 100c as indicated in FIG. 6A. The elongated tubular body 110 can include at least a portion of the treatment lumen 130. The elongated tubular body 110 can include at least a portion of the perfusion lumen 140. The perfusion lumen 140 can further provide a passage way for the guide wire GW as illustrated. Configured as such, the perfusion lumen 140 can include a third opening 150 near the proximal end 112 of the device 100c (See FIG. 1B) to allow the guide wire GW to enter the device 100c. Alternatively, the device 100c can include an additional lumen to allow passage of the guide wire GW; in which case the perfusion lumen 140 need not extend in the proximal direction 12 in relation to the proximal opening 142. The device 100c need not include an inflation lumen.

FIG. 6C illustrates a cross section of the device 100c as indicated in FIG. 6A. The tubular body 110 can be cut away into the perfusion lumen 140 to create the proximal opening 142 of the perfusion lumen 140. The proximal opening 142 of the perfusion lumen 140 can be a skive in the side wall of the tubular body 110.

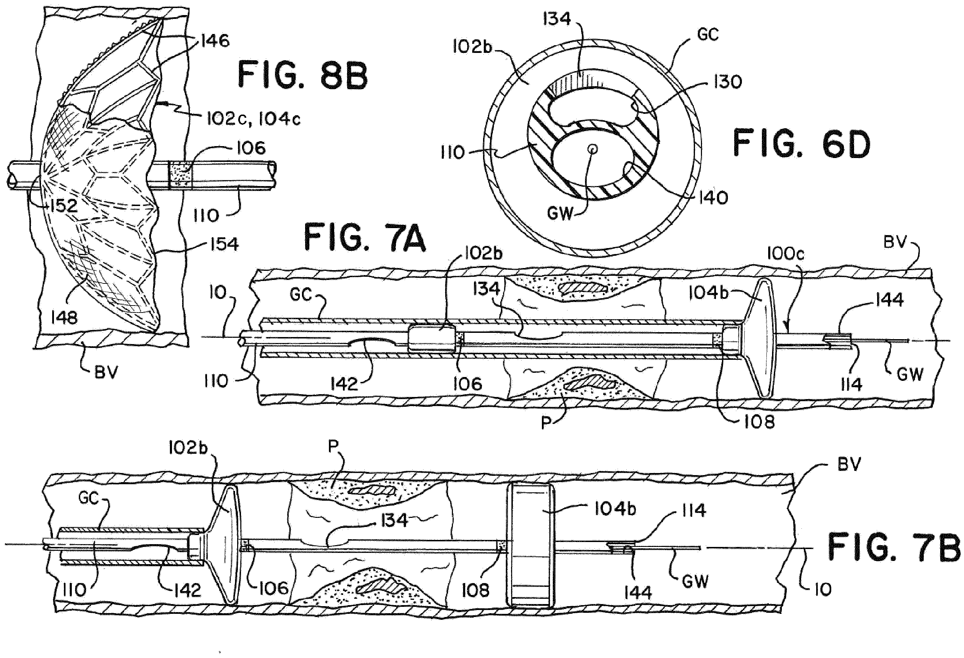

FIG. 6D illustrates a cross section of the device 100c as indicated in FIG. 6A. The tubular body 110 can be cut away into the treatment lumen 130 to create an opening 134 to the treatment lumen 130.

FIGs. 7A through 7C are illustrations of the system illustrated in FIGs. 6A through 6D illustrating the unsheathing of the expandable occlusion elements 102b, 104b.

FIG. 7A illustrates the guide catheter GC moving in the proximal direction 12 while the device 100c remains in position across the lesion P. The distal occluding element 104b can expand to appose the blood vessel BV walls upon being unsheathed. The distal occluding element 104b can include a memory shape material that can move toward a predetermined shape upon heating due to contact with blood. Additionally, or alternatively, the distal occluding element 104b can include a spring-loaded body that is held in compression by the guide catheter GC or other sheath when sheathed and opens when unsheathed.

FIG. 7B illustrates the guide catheter GC moving in the proximal direction 12 while the device 100c remains in position across the lesion P. The distal occluding element 104b can be fully expanded to circumferentially appose the blood vessel BV wall before the proximal expandable element 102b is unsheathed. The proximal occluding element 102b can expand to appose the blood vessel BV walls upon being unsheathed. The proximal occluding element 102b includes, in accordance with the invention, a spring-loaded body that is held in compression by the guide catheter GC when sheathed and opens when unsheathed

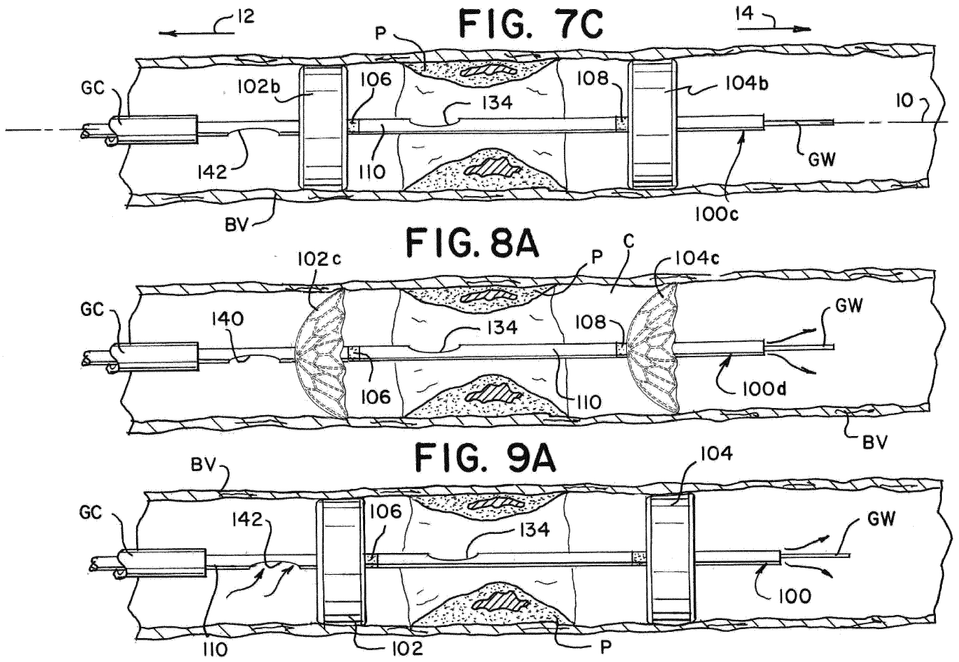

FIG. 7C illustrates the proximal and distal occluding elements 102b, 104b fully expanded. As this step, the cavity C is isolated for treatment.

FIGs. 8A and 8B are illustrations of another system for intravascular treatment functioning according to the principles of the system illustrated in FIGs. 6A through 6D and FIGs. 7A through 7B. The system can include a device 100d having a tube 110 with two self-expandable elements 102c, 104c thereon. The device 100d can further include a treatment lumen 130 and a perfusion lumen 140 such as described in relation to FIGs. 6A through 6D and 7A through 7B.

FIG. 8A illustrates the occluding elements 102c, 104c expanded to occlude a blood vessel BV and isolate a lesion P.

FIG. 8B illustrates component parts of an expandable occluding element 102c, 104c. The expandable occluding element 102c, 104c can include a framework 146 and a coating or cover 148. The coating or cover 148 can be fluid-impermeable. The coating or cover 148 can be flexible such that the coating or cover 148 folds when the expandable occluding element 102c, 104c is in the collapsed configuration. The framework 146 can provide force to appose the occluding element 102c, 104c to the blood vessel BV wall. The framework 146 can include a memory shape material that expands due to heat upon contact with blood. Additionally, or alternatively, the framework 146 can include a spring-loaded material that is held in compression when sheathed and expands when unsheathed. When expanded, the occluding element 102c, 104c can have a parabolic shape having an expanded open end 154 apposing the blood vessel wall and a vertex connected to the tubular body 110. The device 100c can include a connector 152 such as a weld, adhesion, crimp collar, or other connector as appreciated and understood by a person of ordinary skill in the art connecting the vertex of the parabolic shape of the occluding element 102c, 104c to the tubular body 110. The expanded open end 154 can be positioned in the distal direction 14 in relation to the vertex. In other words, the parabolic shape of the occluding element 102c, 104c can expand toward the distal end 114 of the tubular body 110.

FIGs. 9A through 9G are illustrations of steps of treatment using an intravascular treatment system with a treatment device 100 such as illustrated and described in relation to FIGs. 1A and 1B. The treatment device 100 can have a construction and functionality similar to any one of the treatment devices 100a, 100b, 100c, 100d illustrated and described in relation to FIGs. 2A through 2B, 3A through 3G, 4A through 4B, 5A through 5G, 6A through 6D, 7A through 7C, and 8A through 8B, a variation thereof, or an alternative thereto as appreciated and understood by a person of ordinary skill in the art.

FIG. 9A illustrates the device 100 positioned across the lesion P with the occluding elements 102, 104 expanded to isolate the lesion P. The lesion P can be positioned within an intracranial artery. Treatment can include delivering the device 100 to an intracranial artery. The occluding elements 102, 104 and the blood vessel BV wall can define a cavity C containing the lesion P. The cavity C can be positioned in an intracranial artery. The occluding elements 102, 104 can include a balloon such as the occluding elements 102a, 104a illustrated in FIGs. 2A through 2B, 3A through 3G, 4A through 4B, and 5A through 5G. Additionally, or alternatively, the occluding elements 102, 104 can include a self-expanding element such as the occluding elements 102b, 104b, 102c, 104c illustrated in FIGs. 6A through 6D, 7A through 7C and 8A through 8B. The occluding elements can otherwise include a balloon or a self-expanding element as appreciated and understood by a person of ordinary skill in the art. The treatment can include inflating one or both of the occluding elements 102, 104. Additionally, or alternatively, the treatment can include unsheathing one or both of the occluding elements 102, 104 to allow the occluding element(s) to self-expand. The distal occluding element 104 can be expanded before the proximal occluding element 102.

The perfusion lumen 140 can be positioned to serve as a bypass flow tube extending through the proximal occluding element 102, the cavity C, and the distal occluding element 104. The proximal opening 142 of the perfusion lumen 140 can be positioned near the proximal occluding element 102 in the proximal direction 12 in relation to the proximal occluding element 102. The distal opening 144 of the perfusion lumen 140 can be positioned near the distal occluding element 104 in the distal direction 14 in relation to the distal occluding element 104. The distal and proximal openings 144, 142 of the perfusion lumen 140 can be positioned on either side of the lesion P such that when the occluding elements 102, 104 are expanded to define the cavity C, the proximal opening 142 of the perfusion lumen 140 is positioned in the proximal direction 12 in relation to the cavity C and the distal opening 144 of the perfusion lumen is positioned in the distal direction 14 in relation to the cavity C. The proximal and distal openings 142, 144 of the perfusion lumen 140 can be positioned such that neither of the openings 142, 144 is within the cavity C. The perfusion lumen 140 can be isolated from the cavity C. Blood can enter the perfusion lumen 140 at the proximal opening 142 of the lumen 140 (as indicated by the arrows at the proximal opening 142) and exit the perfusion lumen 140 at the distal opening 144 of the lumen 140 (as indicated by the arrows at the distal opening 144) to restore perfusion to the blood vessel BV. In treatments where blood flows oppositely, blood can enter the perfusion lumen 140 at the distal opening 144 and exit the perfusion lumen 140 at the proximal opening 142. Blood can flow through the perfusion lumen 140 without interacting with fluids in the cavity C. The distal opening 134 of the treatment lumen 130 can be positioned within the cavity C. The proximal opening 132 of the treatment lumen 130 can be accessible outside the patient (see FIG. 1B).

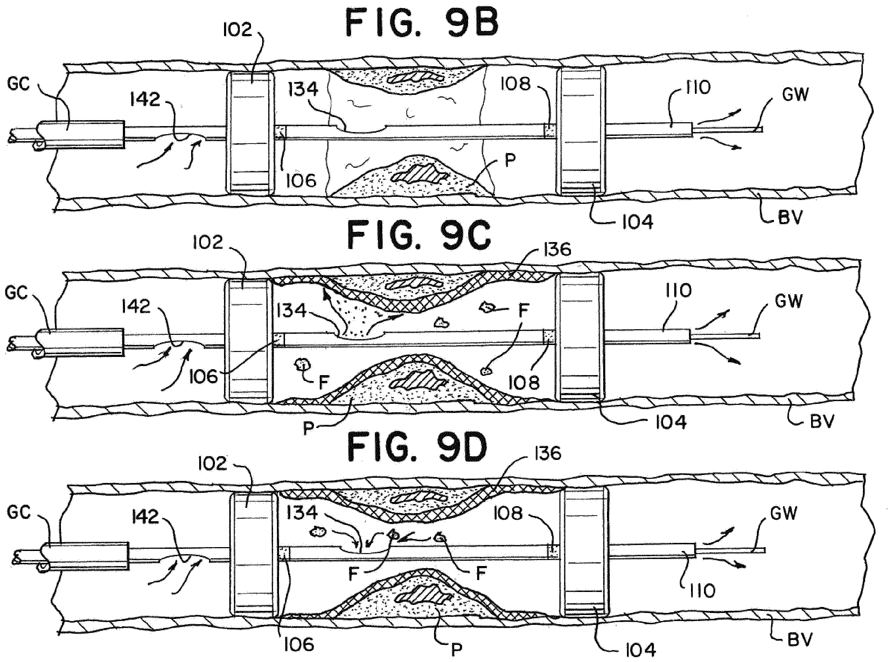

FIG. 9B illustrates a fluid exiting the treatment lumen 130 through the distal opening 134 of the treatment lumen 130 (as indicated by the arrows at the distal opening 134). The treatment can include flowing a fluid through the treatment lumen 130. The fluid can include a drug such as a sealing agent for seating an intravascular lesion. Additionally, or alternatively, the fluid can include a drug effective to disrupt the plaque at the lesion P. The treatment can include delivering a drug to an intracranial artery disease (ICAD) lesion P. Blood can continue to flow through the perfusion lumen 140 while fluid is delivered through the treatment lumen 130.

FIG. 9C illustrates plaque fragments F breaking from the lesion P and floating within the isolated region between the occluding elements 102, 104. Sealant 136 is illustrated coating the lesion P. Blood can continue to flow through the perfusion lumen 140.

FIG. 9D illustrates the fragments F aspirated into the treatment lumen 130 via the distal opening 134 of the treatment lumen 130 (as indicated by arrows at the distal opening 134). Treatment can include aspirating through the treatment lumen 130. Blood can continue to flow through the perfusion lumen 140 during aspiration.

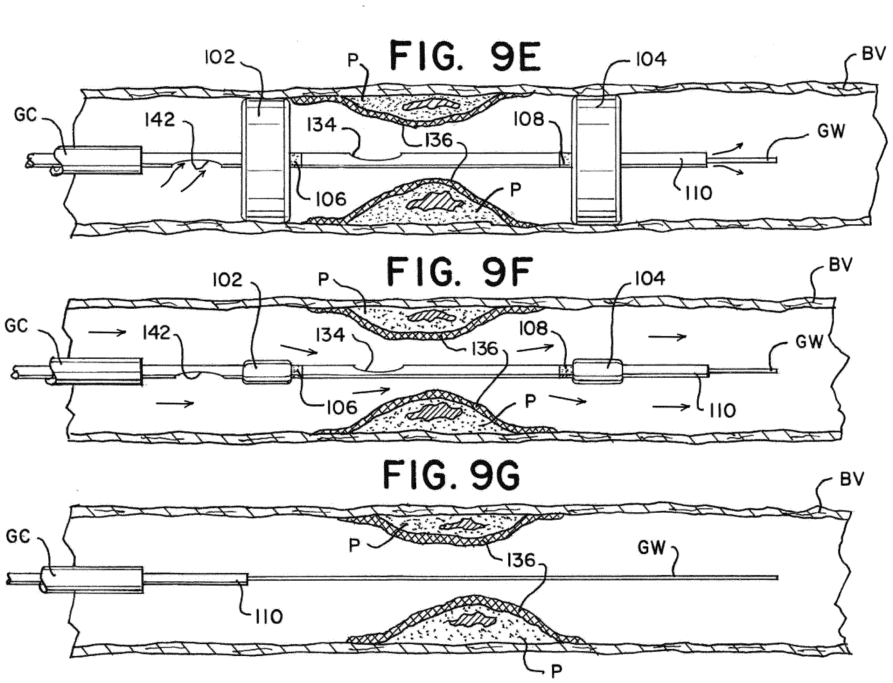

FIG. 9E illustrates blood continuing to flow through the perfusion lumen 140 until the treatment of the lesion is complete. Continued blood flow through the perfusion lumen 140 can facilitate prolonged treatment of the isolated lesion compared to treatments lacking blood perfusion.

FIG. 9F illustrates the collapse of the device 100 for removal from the blood vessel BV. An occluding element including a balloon can be deflated as illustrated. Additionally, or alternatively, an occluding element including a self-expandable element can be collapsed into the guide catheter GC or other sheath.

FIG. 9G illustrates the system being removed from the treatment site.

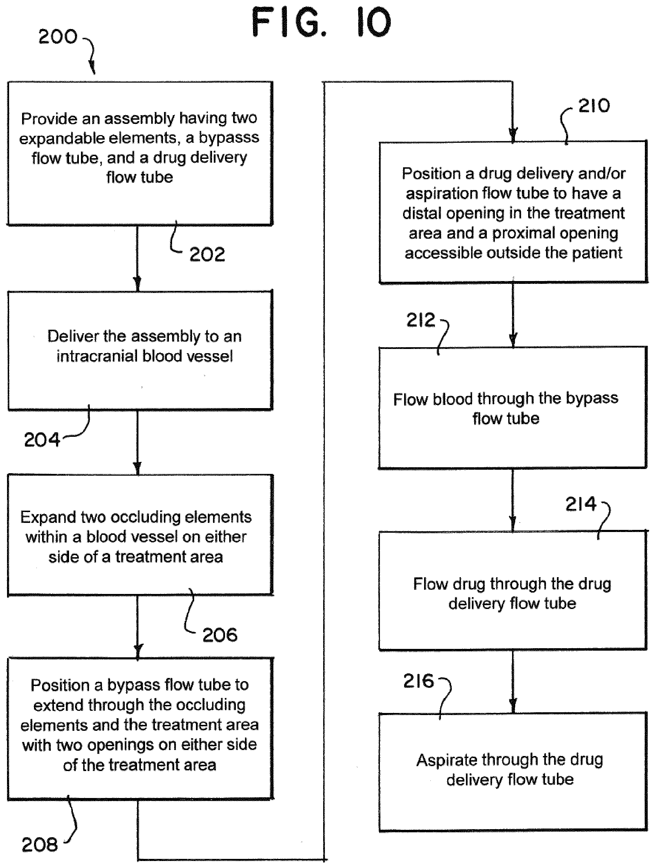

FIG. 10 is a flow diagram of a method 200 for intravascular treatment. The method is not forming part of the claimed invention. In step 202, an assembly having two expandable elements, a bypass flow tube, and a drug delivery flow tube and/or aspiration flow tube can be provided. The two expandable elements can be similar to the expandable elements 102, 102a-c, 104, 104a-c illustrated and described herein, a variation thereof, or an alternative thereto as appreciated and understood by a person of ordinary skill in the art. The bypass flow tube can be similar to the perfusion lumen 140 of the devices 100, 100a-c illustrated and described herein, a variation thereof, or an alternative thereto as appreciated and understood by a person of ordinary skill in the art. The drug delivery flow tube and/or aspiration flow tube can be similar to the treatment lumen 130 of the devices 100, 100a-c illustrated and described herein, a variation thereof, or an alternative thereto as appreciated and understood by a person of ordinary skill in the art.

In step 204, the assembly can be delivered to an intracranial blood vessel. The assembly can be delivered through a guide catheter GC or otherwise delivered as appreciated and understood by a person of ordinary skill in the art.

In step 206, the occluding elements can be expanded within the blood vessel on either side of a treatment area. The occluding elements can be expanded as illustrated and described herein or by other means as appreciated and understood by a person of ordinary skill in the art.

In step 208, the bypass flow tube can be positioned to extend through the occluding elements and the treatment area such that two openings of the bypass flow tube are positioned on either side of the treatment area. The ends of the bypass flow tube can be positioned similar to the positioning of the ends 142, 144 of the perfusion lumen 140 of any of the devices 100, 100a-c illustrated and described herein, a variation thereof, or an alternative thereto as appreciated and understood by a person of ordinary skill in the art.

In step 210, the drug delivery and/or aspiration flow tube can be positioned to have a distal opening at the treatment area and a proximal opening accessible outside the patient. The ends of the drug delivery and/or aspiration flow tube can be positioned similar to the positioning of the ends 132, 134 of the treatment lumen 130 of any of the devices 100, 100a-c illustrated and described herein, a variation thereof, or an alternative thereto as appreciated and understood by a person of ordinary skill in the art.

In step 212, blood can be flowed through the bypass flow tube. Generally speaking, blood can flow through the bypass flow tube at any time when both of the two openings are within a blood vessel. When the blood vessel BV is otherwise blocked (e.g. due to a clot, plaque, and/or expanded expandable elements) between the two openings of the bypass flow tube, blood can be flowed through the bypass flow tube to provide blood perfusion to the blood vessel across the obstructed area.

In step 214, a drug can be flowed through the drug delivery / aspiration flow tube. The drug can include a sealant for sealing an intravascular lesion. Additionally, or alternatively, the drug can include an agent for disrupting plaque.

In step 216, the treatment site can be aspirated through the drug delivery / aspiration flow tube.

The descriptions contained herein are examples of embodiments of the invention and are not intended in any way to limit the scope of the invention.

## Claims

1. A catheter for administering intravascular treatments, the catheter comprising:
an elongated tubular body comprising a proximal end, a distal end, and a length therebetween;
a proximal expandable occluding element (102) disposed on the tubular body, the proximal expandable occluding element expandable from a collapsed shape sized to traverse a guide catheter to an expanded shape sized to occlude a blood vessel;
a distal expandable occluding element (104) disposed on the tubular body in a distal direction in relation to the proximal expandable occluding element, the distal expandable occluding element expandable from a collapsed shape sized to traverse the guide catheter to an expanded shape sized to occlude the blood vessel;
a first fluidic lumen (130) comprising a proximal opening (132) approximate the proximal end of the elongated tubular body and a distal opening (134) positioned in the distal direction in relation to the proximal expandable occluding element and in the proximal direction in relation to the distal expandable occluding element; and
a second fluidic lumen (140) separate from the first fluidic lumen, the second fluidic lumen comprising a proximal opening (142) approximate the proximal expandable occluding element and positioned in the proximal direction in relation to the proximal expandable occluding element and a distal opening (144) approximate the distal expandable occluding element and positioned in the distal direction in relation to the distal expandable occluding element; and
**characterized in that** the proximal expandable occluding element (102) includes a spring-loaded body that is held in compression by the guide catheter when sheathed and opens when unsheathed.

2. The catheter of claim 1,
wherein the elongated tubular body comprises at least a portion of the first fluidic lumen, and
wherein the distal opening of the first fluidic lumen comprises a skive in the elongated tubular body.

3. The catheter of claim 1,
wherein the elongated tubular body comprises at least a portion of the second fluidic lumen, and
wherein the proximal opening of the second fluidic lumen comprises a skive in the elongated tubular body.

4. The catheter of claim 1, wherein the second fluidic lumen provides a contiguous flow path from its proximal opening to its distal opening.

5. The catheter of claim 1,
wherein the first fluidic lumen is sized for delivery of a drug suitable for treating an intravascular lesion, and
wherein the first fluidic lumen is coated with a coating effective to inhibit adherence of a sealant effective to seal an intravascular lesion.

## Patentansprüche

1. Katheter zur Verabreichung von intravaskulären Behandlungen, wobei der Katheter umfasst:
einen langgestreckten rohrförmigen Körper umfassend ein proximales Ende, ein distales Ende und eine Länge dazwischen;
ein proximales expandierbares Absperrelement (102), das an dem rohrförmigen Körper angeordnet ist, wobei das proximale expandierbare Absperrelement von einer kollabierten Form, die zum Durchqueren eines Führungskatheters bemessen ist, zu einer expandierten Form, die zum Absperren eines Blutgefäßes bemessen ist, expandierbar ist;
ein distales expandierbares Absperrelement (104), das an dem rohrförmigen Körper in einer distalen Richtung bezogen auf das proximale expandierbare Absperrelement angeordnet ist, wobei das distale expandierbare Absperrelement von einer kollabierten Form, die zum Durchqueren des Führungskatheters bemessen ist, zu einer expandierten Form, die zum Absperren des Blutgefäßes bemessen ist, expandierbar ist;
ein erstes Fluidlumen (130) umfassend eine proximale Öddnung (132) nahe dem proximalen Ende des langgestreckten rohrförmigen Körpers und eine distale Öffnung (134), die in der distalen Richtung bezogen auf das proximale expandierbare Absperrelement und in der proximalen Richtung bezogen auf das distale expandierbare Absperrelement angeordnet ist; und
ein zweites Fluidlumen (140) getrennt von dem ersten Fluidlumen, wobei das zweite Fluidlumen eine proximale Öffnung (142) nahe dem proximalen expandierbaren Absperrelement und in der proximalen Richtung bezogen auf das proximale expandierbare Absperrelement angeordnet und eine distale Öffnung (144) nahe dem distalen expandierbaren Absperrelement und in der distalen Richtung bezogen auf das distale expandierbare Absperrelement angeordnet umfasst; und
**dadurch gekennzeichnet, dass** das proximale expandierbare Absperrelement (102) einen federbelasteten Körper aufweist, der, wenn umhüllt, durch den Führungskatheter in Kompression gehalten wird und sich bei Freilegen öffnet.

2. Katheter gemäß Anspruch 1,
wobei der langgestreckte rohrförmige Körper wenigstens einen Teil des ersten Fluidlumens umfasst und
wobei die distale Öffnung des ersten Fluidlumens eine Ausfensterung in dem langgestreckten rohrförmigen Körper umfasst.

3. Katheter gemäß Anspruch 1,
wobei der langgestreckte rohrförmige Körper wenigstens einen Teil des zweiten Fluidlumens umfasst und
wobei die proximale Öddnung des zweiten Flzuidlumens eine Ausfensterung in dem langgestreckten rohrförmigen Körper umfasst.

4. Katheter gemäß Anspruch 1, wobei das zweite Fluidlumen einen durchgehenden Flussweg von seiner proximalen Öffnung zu seiner distalen Öffnung bereitstellt.

5. Katheter gemäß Anspruch 1,
wobei das erste Fluidlumen zur Abgabe eines zur Behandlung einer intravaskulären Läsion geeigneten Arzneimittels bemessen ist und
wobei das erste Fluidlumen mit einer Beschichtung beschichtet ist, die wirksam ist, Anhaften eines zum Abdichten einer intravaskulären Läsion geeigneten Dichtmittels zu verhindern.

## Revendications

1. Cathéter pour l'administration de traitements intravasculaires, le cathéter comprenant :
un corps tubulaire allongé comprenant une extrémité proximale, une extrémité distale et une longueur entre celles-ci ;
un élément d'occlusion proximal (102) expansible, disposé sur le corps tubulaire, l'élément d'occlusion proximal expansible étant expansible d'une forme à volume réduit dimensionnée pour traverser un cathéter guide à une forme à volume augmenté dimensionnée pour occlure un vaisseau sanguin ;
un élément d'occlusion distal expansible (104) disposé sur le corps tubulaire dans un sens distal relativement à l'élément d'occlusion proximal expansible, l'élément d'occlusion distal expansible étant expansible d'une forme à volume réduit dimensionnée pour traverser le cathéter guide à une forme à volume augmenté dimensionnée pour occlure le vaisseau sanguin ;
une première lumière à fluide (130) comprenant une ouverture proximale (132) à proximité de l'extrémité proximale du corps tubulaire allongé et une ouverture distale (134) positionnée dans le sens distal relativement à l'élément d'occlusion proximal expansible et dans le sens proximal relativement à l'élément d'occlusion distal expansible ; et
une seconde lumière à fluide (140) séparée de la première lumière à fluide, la seconde lumière à fluide comprenant une ouverture proximale (142) à proximité de l'élément d'occlusion proximal expansible et positionnée dans le sens proximal relativement à l'élément d'occlusion proximal expansible et une ouverture distale (144) à proximité de l'élément d'occlusion distal expansible et positionnée dans le sens distal relativement à l'élément d'occlusion distal expansible ; et
**caractérisé en ce que** l'élément d'occlusion proximal expansible (102) comprend un corps sollicité par ressort qui est maintenu comprimé par le cathéter guide lorsqu'il est gainé et se déploie lorsqu'il est dégainé.

2. Cathéter selon la revendication 1,
dans lequel le corps tubulaire allongé comprend au moins une partie de la première lumière à fluide, et
dans lequel l'ouverture distale de la première lumière à fluide comprend une échancrure dans le corps tubulaire allongé.

3. Cathéter selon la revendication 1,
dans lequel le corps tubulaire allongé comprend au moins une partie de la seconde lumière à fluide, et
dans lequel l'ouverture proximale de la seconde lumière à fluide comprend une échancrure dans le corps tubulaire allongé.

4. Cathéter selon la revendication 1, dans lequel la seconde lumière à fluide définit un chemin d'écoulement contigu de son ouverture proximale à son ouverture distale.

5. Cathéter selon la revendication 1,
dans lequel la première lumière à fluide est dimensionnée pour l'administration d'un médicament approprié pour le traitement d'une lésion intravasculaire, et
dans lequel la première lumière à fluide est pourvue d'un revêtement propre à empêcher l'adhérence d'un produit de scellement propre à sceller une lésion intravasculaire.
